# EUROPEAN PATENT APPLICATION

(11) **EP 3 785 758 A1**
(43) Date of publication of application: **03.03.2021**
(21) Application number: 19194234.1
(22) Date of filing: 29.08.2019
(51) Int. Cl.: A61N 1/05, A61F 2/24, A61N 1/362

(54) **PROSTHETIC HEART VALVE ASSEMBLY COMPRISING A PACEMAKER**

(71) Applicant: BIOTRONIK AG, 8180 Bülach (CH)
(72) Inventor: Skerl, Olaf, 18209 Bad Doberan (DE); Nollert, Georg, 82064 Strasslach (DE)
(74) Representative: Galander, Marcus

(57) **Abstract**

The invention relates to a prosthetic heart valve assembly (1), comprising an expandable frame (2) and at least two heart valve leaflets (4) attached to the expandable frame (2). According to the invention the prosthetic heart valve assembly (1) further comprises a pacemaker (5), the pacemaker (5) comprising a battery, a pacing element (50) and at least one electrode (52, 53), wherein the pacing element (50) is configured to work in VVI mode, wherein the at least one electrode (52, 53) is located in a distal area of the prosthetic heart valve assembly (1) so as to be able to contact a ventricle of a patient's heart if the prosthetic heart valve assembly (1) is implanted into a patient's heart.

## Description

The invention relates to a prosthetic heart valve assembly according to the preamble of claim 1.

In 20% of patients, new, frequently complete atrioventricular blocks (AV blocks) occur after interventional aortic valve replacement (such as transcatheter aortic valve implantation; TAVI). This is due to mechanical pressure on the conduction system or temporary edema at the implant site. In case of self-expanding nitinol stents, an AV block may occur with a delay after implantation so that cardiac arrest may occur in these patients even during the peri-interventional period (e.g., up to 48 hours after TAVI), making pacemaker implantation necessary. Therefore, TAVI patients are currently being treated with a temporary pacemaker probe. Additionally, their electrocardiogram (ECG) is in selected cases monitored for a longer time period (e.g., up to 4 days post TAVI intervention) or these patients are prophylactically treated with a permanent pacemaker. This leads to longer hospital stays, unnecessary costs and complications (e.g., an increased risk of infections such as pneumonia), in particular if elderly or fragile patients are concerned.

The cost for one day of intensive care is more than 1000 €. Temporary pacemaker probes can dislocate and malfunction, leading to cardiovascular arrest. Since the patients are immobile, infections, thromboses, etc. may result.

US 2016/0228250 A1 describes a medical device delivery assembly including a pacing element or including a prosthetic heart valve with one or more pacing elements on it. Therewith, this patent application discloses the possibility of pacing a patient's heart during or after an implantation of a prosthetic heart valve. However, no structural details of the pacing element are disclosed in this patent application.

It is an object of the present invention to provide a low-cost and technically easy implementable possibility to reduce the time of hospital stays after prosthetic heart valve implantation and to reduce the risk of infections of patients having received a prosthetic heart valve (e.g., as in case of a TAVI).

This object is achieved with a prosthetic heart valve assembly having the features according to claim 1. Such a prosthetic heart valve assembly comprises an expandable frame (often also referred to as stent) and at least two heart valve leaflets attached to the frame. Upon expansion of the frame, the at least two heart valve leaflets jointly act together to form an artificial heart valve, i.e., the core of the prosthetic heart valve assembly. Often, in particular in case of a pulmonary valve prosthesis and an aortic valve prosthesis, three valve leaflets are used so as to closely mimic the native pulmonary valve and the native aortic valve.

According to the presently claimed invention, the prosthetic heart valve assembly further comprises a pacemaker. This pacemaker, in turn, comprises a battery, a pacing element and at least one electrode. Thereby, the pacing element is configured to work in VVI mode (Ventricle-Ventricle-Inhibited mode). This means it is configured to stimulate a ventricle of the patient's heart, to sense the signals of a ventricle of the same heart and to work according to inhibition protocol, i.e., to permanently stimulate the heart with a defined frequency, as long as no intrinsic activity of the heart is detected. In case that an intrinsic activity of the heart is detected, the release of stimulating impulses is inhibited. VVI pacemakers can be manufactured with particularly simple electronics and are thus cost-effective pacemakers.

The at least one electrode is located in the distal area of the prosthetic heart valve assembly. The term "distal" is used throughout the present application such as to refer to that part of the prosthetic heart valve assembly that faces towards the ventricle of the patient's heart once the prosthetic heart valve assembly is implanted. Typically, this part of the prosthetic heart valve assembly facing the ventricle is distally arranged on a transcatheter delivery tool used for implanting the prosthetic heart valve assembly into the heart of the patient (with respect to an interventionalist performing the implantation).

Due to its arrangement in a distal area of the prosthetic heart valve assembly, the at least one electrode is able to contact the ventricle of the patient's heart if the prosthetic heart valve assembly is implanted into the patient's heart. Thereby, the concrete location of the at least one electrode on the prosthetic heart valve assembly is typically chosen such that a close contact between the at least one electrode and myocardial tissue of the ventricle of the heart can be established in the implanted state of the prosthetic heart valve assembly.

Patients having received such a prosthetic heart valve assembly can be mobilized and discharged early after TAVI; there is no need for permanent monitoring of patients after TAVI. Unnecessary implantations of a permanent pacemaker with the associated costs and complications after TAVI are no longer necessary; in fact, the risk of AV blocks is declining after resorption of periprocedural edema so that typically no more pacemaker treatment is required after a few days.

In an embodiment, the pacemaker is tightly connected to the expandable frame so that it cannot be detached from the expandable frame. This increases the lifetime of the prosthetic heart valve assembly since no loss of individual parts of the prosthetic heart valve assembly needs to be feared even after a long time period after implantation.

In an embodiment, the at least one electrode is arranged distally at the distal end of the expandable frame. Furthermore, it is connected, either directly or indirectly, to the distal end of the expandable frame in this embodiment. Expressed in other words, the electrode is arranged at an end of the expandable frame which is directed towards the ventricle of the patient's heart when the prosthetic heart valve assembly is implanted into the heart. Then, it is particularly easy to achieve a good electrical contact between the electrode and ventricular myocardium of the patient's heart. Furthermore, in this arrangement, the electrode does not influence the expansion characteristics of the expandable frame to a relevant extent.

In another embodiment, the at least one electrode is arranged on an outside of the expandable frame in a distal end region of the expandable frame. In this embodiment, the electrode is connected, either directly or indirectly, to the expandable frame. In some instances, it might be easier to connect the electrode to the outside of the expandable frame rather than to the distal end of the expandable frame. However, the connection should be made such that the expansion characteristics of the expandable frame are not altered to a significant extent by the applied electrode.

In an embodiment, the pacing element is connected to the at least one electrode by a conductive lead. Thereby, this conductive lead is an element being separate from the struts (or wires) which make up the expandable frame. Expressed in other words, the struts of the frame do not lead any electric energy from the pacing element towards the electrode.

In an embodiment, the conductive lead is tightly attached to at least a part of the struts making up the expandable frame. In another embodiment, the conductive lead is guided over the surface of the expandable frame in a more loosely manner and is able to move within a defined area without significant restrictions over the surface of the expandable frame.

In an embodiment, the pacemaker comprises two electrodes. Thereby, a first electrode serves for sensing ventricular activity of the heart into which the prosthetic heart valve assembly is implanted. In contrast, the second electrode serves for stimulating a ventricle of the heart into which the prosthetic heart valve assembly is implanted. Thus, the pacemaker comprises, in this embodiment, a sensing electrode and a stimulating electrode.

In an embodiment, the sensing electrode and the stimulating electrode are arranged opposite each other on a ring-shaped element which is attached to the distal end of the expandable frame or which is arranged around the circumference of the expandable frame in a distal end region thereof. Then, it is particularly easy to sense ventricular activity at a first ventricular myocardium region and to stimulate the ventricle at a second ventricular myocardium region. In another embodiment, the stimulating electrode and the sensing electrode are arranged closer to each other than in case of lying on opposite sides of a ring-shaped element.

While it is generally possible that the prosthetic heart valve assembly serves for replacing a pulmonary valve, a tricuspid valve or a mitral valve, the prosthetic heart valve assembly serves, in an embodiment, for replacing an aortic heart valve. Thus, it can be denoted as prosthetic aortic valve assembly. This is particularly helpful since the impulse conduction system of the heart is typically particularly impaired in case of an aortic valve replacement. Then, it is particularly helpful if the implanted prosthetic heart valve assembly also comprises a pacemaker that is able to stimulate the patient's heart, if necessary.

The aortic valve prosthesis can be implanted in different ways. One of the best tolerable ways is to implant the aortic valve prosthesis in a transcatheter approach, namely by transcatheter aortic valve implantation (TAVI; also referred to with the abbreviations PAVR or TAVR in certain instances; all of which may be used interchangeably). Therefore, the prosthetic aortic valve assembly is, in an embodiment, configured to be implanted into a patient by a TAVI (PAVR or TAVR) procedure.

Typically, AV blocks occur only in a small time period directly or shortly after the implantation of a heart valve prosthesis like the prosthetic heart valve assembly, in particular prosthetic aortic valve assembly, as explained in the present application. Therefore, pacing of the patient needs only to occur within a few days after the prosthesis implantation (e.g., within 24 hours, 48 hours, 72 hours or 96 hours after implantation). Thus, it is not necessary to equip the pacemaker of the prosthetic heart valve assembly with a battery having a capacity for more than 6 months. Rather, in an embodiment, the battery of the pacemaker has a capacity which is sufficient to provide the pacing element with energy required for pacing the patient's heart over a time period of up to 7 to 180 days, in particular up to 30 to 150 days, in particular up to 60 days. After that time period, the pacemaker can simply remain on the expandable frame and is no longer needed for pacing activities. A battery having such a capacity may serve for safe pacing the patient's heart, if required, within the typically required timeframe of a couple of days or a couple of months, yet exhibiting sufficient safety buffer to ensure proper functioning of the pacemaker in the required time period.

In an embodiment, the battery is a solid-state battery, in particular a solid-state battery of compact design. Then, the battery requires only little space and can be placed on an outside of the expandable stent without significantly altering the overall properties of the prosthetic heart valve assembly.

In an embodiment, the pacemaker is activated prior to implantation of the prosthetic heart valve assembly. In another embodiment, the pacemaker is activated from external of the patient's body after implantation of the prosthetic heart valve assembly. In a specific embodiment, the pacemaker is configured to be automatically activated upon deployment of the expandable frame during implantation of the prosthetic heart valve assembly. Then, no manual activation either prior to or after implantation is necessary. Rather, the activation step is automatically done during implantation.

In an aspect, the present invention relates to a method of treatment of a patient in need thereof with ventricular pacing signals. This is done in order to achieve a ventricular contraction of the patient's heart. Thereby, the ventricular pacing signals are provided by a pacemaker. This pacemaker comprises a battery, a pacing element and at least one electrode. Thereby, the pacemaker forms part of the prosthetic heart valve assembly that additionally comprises an expandable frame (also referred to as expandable stent) and at least two heart valve leaflets attached to the frame. The pacing element is configured to work in VVI mode. Furthermore, the at least one electrode is located in a distal area of the prosthetic heart valve assembly and contacts the ventricle of the patient's heart in an implanted state of the prosthetic heart valve assembly. Due to this contact, pacing signals can be particularly well introduced by the at least one electrode into the myocardium of the patient.

In an embodiment, the method is carried out such that the ventricular pacing signals are provided to the patient within a time period of up to 7 to 180 days after implantation of the prosthetic heart valve assembly, in particular up to 30 to 150 days, in particular up to 60 days.

In an embodiment, the method is carried out such that the ventricular pacing signals are provided to the patient within a time period of less than 10 days after implantation of the prosthetic heart valve assembly, in particular up to 5 days, in particular up to 72 hours, in particular up to 48 hours, in particular within 24 hours after implantation of the prosthetic heart valve assembly.

All embodiments of the described prosthetic heart valve assembly can be combined in any desired way and can be transferred either alone or in any combination to the described method of treatment, and vice versa.

Further details of aspects of the present invention will be explained in the following with respect to exemplary embodiments and corresponding Figures. In the Figures:
- Fig. 1: shows a schematic depiction of an embodiment of a prosthetic heart valve assembly in its implanted state, and
- Fig. 2: shows an exemplary embodiment of a circuit diagram of a pacemaker working in VVI mode.

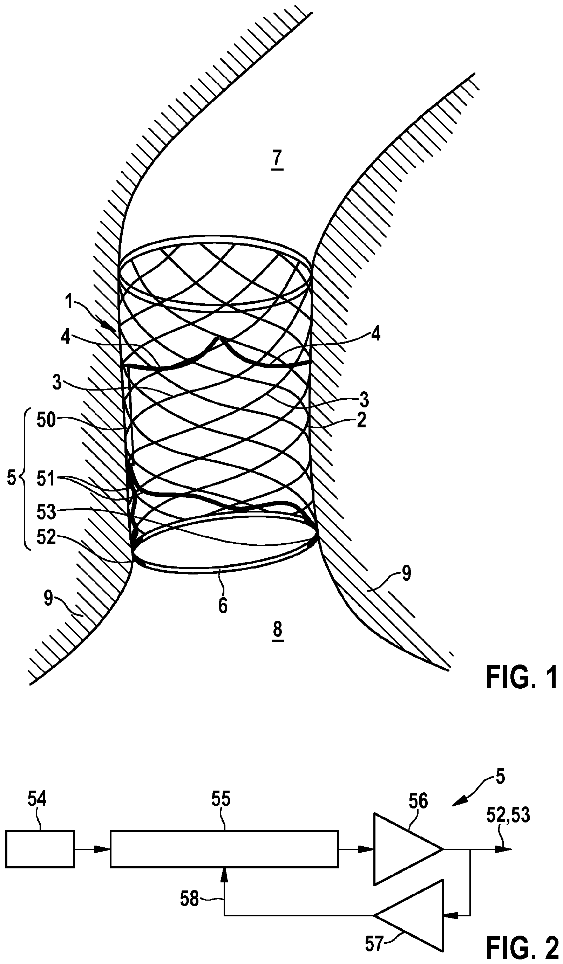

Figure 1 shows a schematic depiction of an aortic valve prosthesis 1 serving as prosthetic heart valve assembly. This aortic valve prosthesis 1 comprises an expandable stent 2 acting as expandable frame. This expandable stent 2 is made up of a plurality of struts 3, wherein only some of these struts 3 are marked with the corresponding numeral reference.

Furthermore, the aortic valve prosthesis 1 comprises three valve leaflets 4, two of which are visible in the depiction of Figure 1.

Exemplarily, on an outside of the expandable stent 2, a pacing element 50 (including a battery which is not visible in the depiction of Figure 1) is arranged which is connected via two conductive leads 51 to a first electrode 52 and a second electrode 53. The first electrode 52 and the second electrode 53 are arranged on a ring-shaped element 6 at opposite sides thereof. Thereby, the ring-shaped element 6 is connected to the distal end of the expandable stent 2. The pacing element 50, the conductive leads 51, the first electrode 52 and the second electrode 53 make up together a pacemaker 5.

The aortic valve prosthesis 1 is depicted in Figure 1 in its implanted state arranged between an aortic lumen 7 and the ventricle 8 of the patient's heart. Thereby, it is located at a site at which the natural aortic valve is placed. Therefore, the first electrode 52 and the second electrode 53 are able to contact ventricular myocardium 9 of the patient.

The pacing element 50 of the pacemaker 5 works in VVI mode. I.e., it has a fixed stimulation interval that can be set during manufacturing of the aortic valve prosthesis 1 or prior to implantation of the aortic valve prosthesis 1. The stimulation interval can be, e.g., 1.2 seconds (corresponding to a heart rate of 50 beats per minute (bpm)). If either the first electrode 52 or the second electrode 53 senses a ventricular activity prior to the termination of the stimulation interval, no stimulation will take place, i.e., the stimulation is inhibited. However, if no ventricular activity is sensed up to the end of the stimulation interval, automatic stimulation of the ventricle takes place. This prevents a life-threatening bradycardia.

The pacing element 50 comprises the necessary circuitry for sensing ventricular activity and for stimulating the ventricle of the patient, if necessary. It comprises only a very small solid-state battery since its functioning needs to be guaranteed only over a period of approximately 90 days, wherein ventricular stimulation after implantation of the aortic heart valve prosthesis is typically only necessary within 72 hours after implantation if AV blocks occurred.

Figure 2 shows a circuit diagram of an embodiment of a VVI circuit that can be implemented in the pacemaker 5 of Figure 1. Similar elements are denoted with the same numeral references as in Figure 1.

The pacemaker 5 comprises a clock 54 that defines a counting pulse for a timer 55. The timer 55 is set to a fixed time, e.g., 1.2 seconds (corresponding to a heart rate of 50 bpm). When the timer 55 reaches the set time, a stimulation pulse is generated by a pace output 56 and transferred to the first electrode 52 and/or the second electrode 53.

The first electrode 52 and/or the second electrode 53 are also connected with a sense amplifier 57. If the sense amplifier 57 senses during the set time interval of the timer 55 any ventricular activity, the timer is reset by a reset command 58. Consequently, no stimulation impulse will be generated by the pace output 56. Rather, such a stimulation impulse is only generated by the pace output 56, if no ventricular activity is sensed up to the end of the set time, i.e., if the heart rate is lower than, e.g., 50 bpm. If the heart rate is constantly higher than, e.g., 50 bpm, no stimulation is carried out at all by the pacemaker 5.

While Figure 2 illustrates one embodiment of a circuitry being able to apply pacing in VVI mode, it should be fully understood that also other implementations of a VVI mode can be adapted by the pacing element 50 of the pacemaker 5.

In view of all the foregoing disclosure, further embodiments of the present invention are reflected by the following consecutively numbered embodiments:
1. A prosthetic heart valve assembly, comprising an expandable frame (2) and at least two heart valve leaflets (4) attached to the expandable frame (2),
   **characterized in that**
   the prosthetic heart valve assembly (1) further comprises a pacemaker (5), the pacemaker (5) comprising a battery, a pacing element (50) and at least one electrode (52, 53), wherein the pacing element (50) is configured to work in VVI mode, wherein the at least one electrode (52, 53) is located in a distal area of the prosthetic heart valve assembly (1) so as to be able to contact a ventricle of a patient's heart if the prosthetic heart valve assembly (1) is implanted into a patient's heart.
2. The prosthetic heart valve assembly according to embodiment 1, **characterized in that** the pacemaker (5) is connected to the expandable frame (2) in a non-detachable manner.
   In this context, the expression "non-detachable manner" means that the pacemaker is fixedly connected to the expandable frame.
3. The prosthetic heart valve assembly according to embodiment 1 or 2, **characterized in that** the at least one electrode (52, 53) is arranged distally of a distal end of the expandable frame (2) and is connected to the distal end of the expandable frame (2).
4. The prosthetic heart valve assembly according to embodiment 1 or 2, **characterized in that** the at least one electrode (52, 53) is arranged on an outside of the expandable frame (2) in a distal end region of the expandable frame (2) and is connected to the expandable frame (2).
5. The prosthetic heart valve assembly according to any one of the preceding embodiments, **characterized in that** the pacing element (50) is connected to the at least one electrode (52, 53) via a conductive lead (51) being distinct from struts (3) of the expandable frame.
6. The prosthetic heart valve assembly according to any one of the preceding embodiments, **characterized in that** the pacemaker (5) comprises two electrodes (52, 53), wherein a first electrode (52) serves for sensing ventricular activity of a heart into which the prosthetic heart valve assembly (1) is implanted and a second electrode (53) serves for stimulating a ventricle of a heart into which the prosthetic heart valve assembly (1) is implanted.
7. The prosthetic heart valve assembly according to any one of the preceding embodiments, **characterized in that** the prosthetic heart valve assembly (1) is a prosthetic aortic valve assembly.
8. The prosthetic heart valve assembly according to embodiment 7, **characterized in that** the prosthetic aortic valve assembly (1) is configured to be implanted into a patient's heart by a TAVI, PAVR, or TAVR procedure.
   With this context, the abbreviation "TAVI" denotes transcatheter aortic valve implantation, the abbreviation "PAVR" denotes percutaneous aortic valve replacement, and the abbreviation "TAVR" denotes transcatheter aortic valve replacement; all of which abbreviations may be used interchangeably with the context of the present invention.
9. The prosthetic heart valve assembly according to any one of the preceding embodiments, **characterized in that** the battery has a capacity sufficient to provide the pacing element (50) with energy required for pacing a patient's heart over a time period of up to 60 to 180 days.
10. The prosthetic heart valve assembly according to any one of the preceding embodiments, **characterized in that** the battery is a solid state battery.
11. The prosthetic heart valve assembly according to any one of the preceding embodiments, **characterized in that** the pacemaker (5) is configured to be automatically activated upon deployment of the expandable frame (2) during implantation of the prosthetic heart valve assembly (1).
12. A method of treatment of a patient in need thereof with ventricular pacing signals in order to achieve a ventricular contraction of the patient's heart, wherein the ventricular pacing signals are provided by a pacemaker, the pacemaker comprising a battery, a pacing element and at least one electrode, the pacemaker forming part of a prosthetic heart valve assembly, additionally comprising an expandable frame and at least two heart valve leaflets attached to the frame, wherein the pacing element is configured to work in VVI mode, wherein the at least one electrode is located in a distal area of the prosthetic heart valve assembly and contacts a ventricle of the patient's heart in an implanted state of the prosthetic heart valve assembly.
13. The method of treatment according to embodiment 12, wherein the ventricular pacing signals can be provided to the patient within a time period of up to 7 to 180 days after implantation of the prosthetic heart valve assembly, in particular within a time period of up to 72 hours after implantation of the prosthetic heart valve assembly.

## Claims

1. A prosthetic heart valve assembly, comprising an expandable frame (2) and at least two heart valve leaflets (4) attached to the expandable frame (2),
**characterized in that**
the prosthetic heart valve assembly (1) further comprises a pacemaker (5), the pacemaker (5) comprising a battery, a pacing element (50) and at least one electrode (52, 53), wherein the pacing element (50) is configured to work in VVI mode, wherein the at least one electrode (52, 53) is located in a distal area of the prosthetic heart valve assembly (1) so as to be able to contact a ventricle of a patient's heart if the prosthetic heart valve assembly (1) is implanted into a patient's heart.

2. The prosthetic heart valve assembly according to claim 1, **characterized in that** the pacemaker (5) is connected to the expandable frame (2) in a non-detachable manner.

3. The prosthetic heart valve assembly according to claim 1 or 2, **characterized in that** the at least one electrode (52, 53) is arranged distally of a distal end of the expandable frame (2) and is connected to the distal end of the expandable frame (2).

4. The prosthetic heart valve assembly according to claim 1 or 2, **characterized in that** the at least one electrode (52, 53) is arranged on an outside of the expandable frame (2) in a distal end region of the expandable frame (2) and is connected to the expandable frame (2).

5. The prosthetic heart valve assembly according to any one of the preceding claims, **characterized in that** the pacing element (50) is connected to the at least one electrode (52, 53) via a conductive lead (51) being distinct from struts (3) of the expandable frame.

6. The prosthetic heart valve assembly according to any one of the preceding claims, **characterized in that** the pacemaker (5) comprises two electrodes (52, 53), wherein a first electrode (52) serves for sensing ventricular activity of a heart into which the prosthetic heart valve assembly (1) is implanted and a second electrode (53) serves for stimulating a ventricle of a heart into which the prosthetic heart valve assembly (1) is implanted.

7. The prosthetic heart valve assembly according to any one of the preceding claims, **characterized in that** the prosthetic heart valve assembly (1) is a prosthetic aortic valve assembly.

8. The prosthetic heart valve assembly according to claim 7, **characterized in that** the prosthetic aortic valve assembly is configured to be implanted into a patient's heart by a TAVI, PAVR, or TAVR procedure.

9. The prosthetic heart valve assembly according to any one of the preceding claims, **characterized in that** the battery has a capacity sufficient to provide the pacing element (50) with energy required for pacing a patient's heart over a time period of up to 60 to 180 days.

10. The prosthetic heart valve assembly according to any one of the preceding claims, **characterized in that** the battery is a solid state battery.

11. The prosthetic heart valve assembly according to any one of the preceding claims, **characterized in that** the pacemaker (5) is configured to be automatically activated upon deployment of the expandable frame (2) during implantation of the prosthetic heart valve assembly (1).

12. A method of treatment of a patient in need thereof with ventricular pacing signals in order to achieve a ventricular contraction of the patient's heart, wherein the ventricular pacing signals are provided by a pacemaker, the pacemaker comprising a battery, a pacing element and at least one electrode, the pacemaker forming part of a prosthetic heart valve assembly, additionally comprising an expandable frame and at least two heart valve leaflets attached to the frame, wherein the pacing element is configured to work in VVI mode, wherein the at least one electrode is located in a distal area of the prosthetic heart valve assembly and contacts a ventricle of the patient's heart in an implanted state of the prosthetic heart valve assembly.

13. The method of treatment according to claim 12, wherein the ventricular pacing signals can be provided to the patient within a time period of up to 7 to 180 days after implantation of the prosthetic heart valve assembly, in particular within a time period of up to 72 hours after implantation of the prosthetic heart valve assembly.
